# EUROPEAN PATENT SPECIFICATION

(11) **EP 3 850 547 B1**
(45) Date of publication and mention of the grant of the patent: **21.02.2024**
(21) Application number: 19863699.5
(22) Date of filing: 16.09.2019
(51) Int. Cl.: G06K 19/077, C12Q 1/68, C12Q 1/6806

(54) **SYSTEM AND METHOD FOR THE PRODUCTION AND TREATMENT OF FUR, SKIN, AND LEATHER COMMODITIES**
SYSTEM UND VERFAHREN ZUR HERSTELLUNG UND BEHANDLUNG VON PELZ-, HAUT- UND LEDERWAREN
SYSTÈME ET PROCÉDÉ DE PRODUCTION ET DE TRAITEMENT DE PRODUITS DE FOURRURE, DE PEAU ET DE CUIR

(30) Priority: 17.09.2018 US 201862732289 P
(43) Date of publication of application: 21.07.2021
(73) Proprietor: PIEL.com S.A., Panama (PA)
(72) Inventor: RUBMAN, Scott, George Town (KY)
(74) Representative: Page White Farrer
(86) International application number: PCT/US2019/051313
(87) International publication number: WO 2020/060928

(56) References cited:
- WO-A1-2018/094357
- KR-A- 20130 133 123
- US-A1- 2015 140 058
- US-A1- 2015 273 737
- US-A1- 2016 137 863
- US-A1- 2017 243 284
- US-A1- 2017 243 284

## Description

### CROSS REFERENCE TO RELATED APPLICATION

This application claims the benefit of U.S. Provisional Patent Application No. 62/732,289, filed on September 17, 2018.

### FIELD OF INVENTION

The present disclosure is directed to a system and method of treating a portion of a fur, skin, or leather commodity to facilitate identification. In another embodiment, the present disclosure is directed to a system and method of producing fur, skin, or leather commodities.

### BACKGROUND

The business-to-business side of the fur industry involves the purchase of fur pelts for the manufacture of garments or other articles. Likewise, the business-to-business side of the leather industry involves the purchase of leather skins for the manufacture of garments or articles. Fur pelts and leather skins are typically available in limited supply from fur farmers, ranchers, or trappers, and may be of varying quality. A purchaser may determine the quality of the pelts or skins through personal inspection, or through verification by a trusted seller or intermediary. Therefore, the business-to-business side of the fur and leather industries is primarily conducted in a face-to-face auction environment on limited occasions. Such auctions require purchasers to travel to locations throughout the world to procure raw product. Alternatively, the purchaser may engage an agent or purchase pelts or skins from a broker (or a chain of intermediaries) that purchased the pelts or skins through an auction or a private treaty with particular farmers, ranchers, or trappers. A manufacturer may also purchase pelts or skins directly from a farmer, rancher, or trapper.

The manufacturer then manufactures garments or other articles and may sell the articles to department stores, independent stores, or leased facilities. The manufacturer may sell the articles through a supply chain of distributors. Alternatively, the manufacturer may sell directly to consumers. The consumer can determine the quality and authenticity of the garment through a visual inspection or by trusting the representations of the seller.

Prior patent applications, including U.S. Provisional Patent Application No. 62/299,096, U.S. Patent Application No. 15/441,755, and International Patent Application No. PCT/US2017/047774 are directed to a platform and data repository for fur, skin, or feather commodities (such as leather skins and other hides or skins, including reptile skin, fish skin, ostrich skin, and bird feathers). More particularly, those applications are directed to a platform and repository that provide for the digital identification and the adjudication of the provenance of fur pelts and articles or leather goods based upon and backed up by DNA.

United States Patent application Publication number US 2017/243284 A1 relates to a method of tracking a provenance of a fur or a leather skin includes providing at least one of a live animal, a fur pelt, and a leather skin, removing a DNA sample from the at least one of the live animal, the fur pelt, and the leather skin, and storing the DNA sample. The DNA sample and DNA information is stored in a DNA repository that may be securely accessed by authorized individuals through a networked device, such as a computer, smart phone, or tablet. The method further includes assigning a globally unique identifier number to the at least one of the live animal, the fur pelt, and the leather skin and storing the identifier number. The method further includes selling the at least one of the live animal, the fur pelt, and the leather skin and storing sale information and associating the sale information with at least one of the identifier number and the DNA sample.

United States Patent application Publication number US 2015/273737 A1 relates to a flexible and stretchable graphene film, comprising a graphene layer and a functional layer formed on the graphene layer; a preparing method of the graphene film. This US patent application further relates to a method of producing a graphene film, comprising: (a) providing a carrier, and a graphene layer is formed on the surface of the carrier, (b) forming a functional layer containing an insulating polymer and a conductive material on a top portion of the graphene film; and (c) dissolving the carrier by an etchant.

To facilitate identification of fur, skin, or feather commodities, a need exists to protect at least a portion of the commodity during chemical treatments to preserve DNA within that commodity. A need further exists to mark or tag fur, skin, or feather commodities while preserving the look, feel, and quality of the commodity. Commodities that have been protected or marked may be identified and tracked in an above described platform and data repository. However, such protection and marking may be done independently of any platform or data repository.

Additionally, a need exists to produce fur, skin, or feather commodities independently of a living animal.

### SUMMARY OF THE INVENTION

The present invention is defined by the appended independent claims. Certain more specific aspects are defined by the dependent claims.

In one embodiment, a semiconductor material is applied to a portion of a fur, skin, or feather commodity. The material is applied in a liquid, aerosol, or adhesive formulation, such that the material is molecularly adhered to (or adsorbed by) the fur, skin, or feather commodity. Alternatively, the material may be applied by a laser burning or branding process. The material may be applied prior to a chemical treatment of the fur, skin, or feather commodity.

In another embodiment, a molecular adsorbing system is capable of transmitting or receiving digital certification information in either a liquid, aerosol or adhesive format. The material may include but is not limited to graphene.

In another embodiment, a fur, skin, or feather commodity is produced by an additive manufacturing process. In one such embodiment, skin is created through a 3D bioprinting process. Hair or feathers are then grafted onto the bioprinted skin.

### BRIEF DESCRIPTION OF THE DRAWINGS

In the accompanying drawings, structures are illustrated that, together with the detailed description provided below, describe exemplary embodiments of the claimed invention. Like elements are identified with the same reference numerals. It should be understood that elements shown as a single component may be replaced with multiple components, and elements shown as multiple components may be replaced with a single component. The drawings are not to scale and the proportion of certain elements may be exaggerated for the purpose of illustration.
**Figure 1** is a schematic drawing illustrating the application of a material to a skin, feather, or fur commodity; and
**Figure 2** is a schematic drawing illustrating a bioprinting system and process.

### DETAILED DESCRIPTION

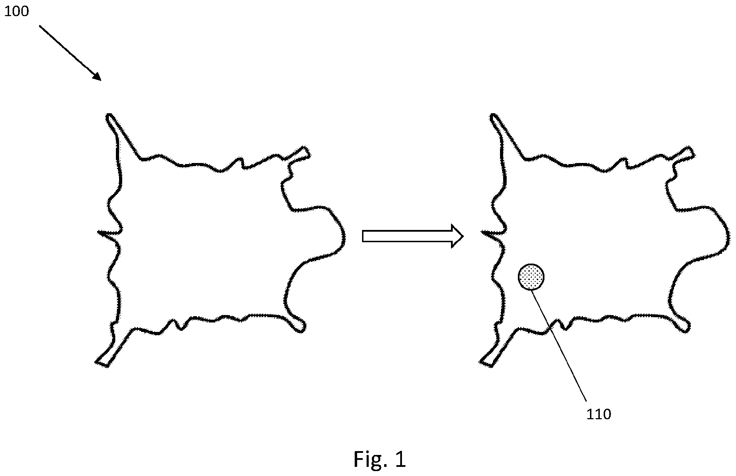

**Figure 1** is a schematic drawing illustrating the application of a material to a skin or fur commodity. In the illustrated embodiment, a fur commodity **100** is provided. In alternative embodiments, a skin (such as leather skins or other hides or skins, including reptile, fish and ostrich skin) or feather commodity may be provided. While the process below is described in the context of a fur commodity, it should be understood that the process may be similarly employed for a skin or feather commodity.

A material **110** is applied to at least a portion of the fur commodity **100.** While **Figure 1** shows a visible, circular region of material **110,** it should be understood that this is merely for illustrative purposes. The material may be applied to a polygonal or irregular shaped region of the fur commodity. The region may be small, so as not to be easily noticeable or even visible at all. Alternatively, the region may be larger, even encompassing the entire fur commodity.

The material **110** may be applied to the fur commodity **100** in several different ways. In one embodiment, the material **110** is applied in a solid form or in a liquid state. such that the material is molecularly adhered to (or adsorbed by) the fur, skin, or feather commodity. For example, the material **110** aerosol or adhesive formulation. Alternatively, the material **110** may be applied by a laser burning or branding process.

In one embodiment, the material **110** is a conductive material capable of storing electricity, and thus capable of storing digital information. The material **110** may be a semi-metal or other semiconductor material. In one specific embodiment, the material is a zero-gap semiconductor. Exemplary properties of the material **110** may include, without limitation, a valley degeneracy of gv = 2, electron mobility greater than 15,000 cm² × V⁻¹ × s⁻¹, and resistivity of less than 10⁻⁶ ohm-cm. One specific example of the material **110** is graphene. However, it should be understood that the material may be a non-metal.

The material **110** may be selected such that is molecularly adhered to (or adsorbed by) the fur commodity **100.** In other words, the material **110** adheres to the surface of the fur commodity **100** without dissolving or permeating into the fur commodity **100.** Thus, the material **110** forms a protective layer over the portion of the fur commodity **100,** and may preserve underlying DNA during any chemical, thermal, or other treatment of the fur commodity. Thus, if DNA testing of the fur commodity **100** is desired at a later time, the material **110** may be removed and DNA may be extracted.

The electrical properties of the material **110** may also allow the transmission and storage of digital information. Digital information, such as digital certification information may be embedded into the material **110** and may be transmitted through a scanning process. Thus, the fur commodity **100** and material **110** may be characterized as a molecular adsorbing system that is capable of transmitting or receiving digital certification information in either a liquid, aerosol or adhesive format.

In some embodiments, the material **110** is not capable of storing digital information, but still protects the fur commodity **100** from experiencing DNA damage. In such an embodiment, the material **110** may be used in conjunction with an RFID or other device capable of storing and transmitting data.

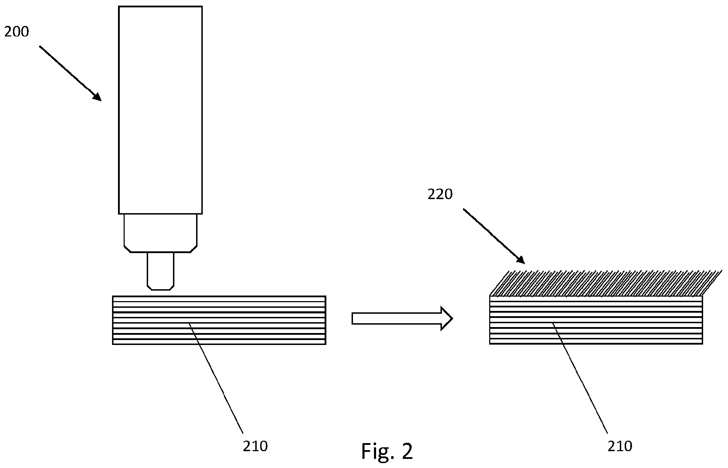

**Figure 2** is a schematic drawing illustrating a bioprinting process. A 3D bioprinter **200** extrudes layers of biomaterials and living cells **210** to make biometric skin. The skin cells may include, without limitation, melanocytes, keratinocytes, and fibroblasts. Alternatively, or in addition, stem cells may be employed. Multiple layers of biomaterials and living cells may be overlaid until a biometric skin of desired thickness has been produced.

After the biometric skin is produced, hair or feathers are shorn from an animal and grafted onto the biometric skin. The hair or feathers may also be grated or planted onto the biometric skin, similar to a process of planting seeds in soil. The hair or feathers then grow on the biometric skin.

In one embodiment, the biomaterials and living cells used to produce the biometric skin are from the same animal that is shorn of hair or feathers. In another embodiment, the biomaterials and living cells used to produce the biometric skin are from a different animal that is shorn of hair or feathers. In this embodiment, the animals may be from the same species or from different species.

In an alternative embodiment, synthetic skin is produced without living cells.

While the present disclosure has been illustrated by the description of embodiments thereof, and while the embodiments have been described in considerable detail, it is not the intention of the applicants to restrict or in any way limit the scope of the appended claims to such detail. Additional advantages and modifications will readily appear to those skilled in the art. Therefore, the disclosure, in its broader aspects, is not limited to the specific details, the representative apparatus and method, and illustrative examples shown and described. Accordingly, departures may be made from such details without departing from the scope of the applicant's general inventive concept.

To the extent that the term "includes" or "including" is used in the specification or the claims, it is intended to be inclusive in a manner similar to the term "comprising" as that term is interpreted when employed as a transitional word in a claim. Furthermore, to the extent that the term "or" is employed (e.g., A or B) it is intended to mean "A or B or both." When the applicants intend to indicate "only A or B but not both" then the term "only A or B but not both" will be employed. Thus, use of the term "or" herein is the inclusive, and not the exclusive use. See, Bryan A. Garner, A Dictionary of Modern Legal Usage 624 (2d. Ed. 1995). Also, to the extent that the terms "in" or "into" are used in the specification or the claims, it is intended to additionally mean "on" or "onto." Furthermore, to the extent the term "connect" is used in the specification or claims, it is intended to mean not only "directly connected to," but also "indirectly connected to" such as connected through another component or components.

## Claims

1. A method of testing DNA of a fur, skin, or feather commodity, the method comprising:
applying a semiconductor material to a portion of a fur, skin, or feather commodity, thereby forming a protective layer against chemical treatments and defining a protected portion of the fur, skin, or feather commodity;
chemically treating the fur, skin, or feather commodity;
removing the semiconductor material from the protected portion of the fur, skin, or feather commodity;
extracting DNA from the protected portion of the fur, skin, or feather commodity; and
testing the extracted DNA.

2. The method of claim 1, wherein the semiconductor material is applied such that the material is molecularly adhered to the fur, skin, or feather commodity.

3. The method of claim 1 or 2, wherein the semiconductor material has a valley degeneracy of gv = 2.

4. The method of any one of claims 1-3, wherein the semiconductor material has electron mobility greater than 15,000 cm² × V⁻¹ × s⁻¹.

5. The method of any one of claims 1-4, wherein the semiconductor material has resistivity of less than 10⁻⁶ ohm-cm.

6. The method of any one of claims 1-5, wherein the semiconductor material is graphene.

7. The method of any one of claims 1-6, wherein the semiconductor material is applied as an adhesive formulation.

8. A chemically treated fur, skin, or feather commodity comprising:
a fur, skin, or feather commodity; and
a semiconductor material molecularly adhered to a portion of the fur, skin, or feather commodity, thereby providing a protective layer against chemical treatments and defining a protected portion of the fur, skin, or feather commodity.

9. The treated fur, skin, or feather commodity of claim 8, wherein the semiconductor material is a zero-gap semiconductor.

10. The treated fur, skin, or feather commodity of claim 8 or 9, wherein the semiconductor material has a valley degeneracy of gv = 2.

11. The treated fur, skin, or feather commodity of any one of claims 8-10, wherein the semiconductor material has electron mobility greater than 15,000 cm² × V⁻¹ × s⁻¹.

12. The treated fur, skin, or feather commodity of any one of claims 8-11, wherein the semiconductor material has resistivity of less than 10⁻⁶ ohm-cm.

13. The treated fur, skin, or feather commodity of any one of claims 8-12, wherein the semiconductor material includes digital information stored thereon.

## Patentansprüche

1. Verfahren zum Testen von DNA eines Pelz-, Haut- oder Federartikels, wobei das Verfahren aufweist:
Aufbringen eines Halbleitermaterials auf einen Teil eines Pelz-, Haut- oder Federartikels, wodurch eine Schutzschicht gegen chemische Behandlungen gebildet wird und ein geschützter Teil des Pelz-, Haut- oder Federartikels definiert wird;
chemisches Behandeln des Pelz-, Haut- oder Federartikels;
Entfernen des Halbleitermaterials aus dem geschützten Teil des Pelz-, Haut- oder Federartikels;
Extrahieren von DNA aus dem geschützten Teil des Pelz-, Haut- oder Federartikels; und
Testen der extrahierten DNA.

2. Verfahren nach Anspruch 1, wobei das Halbleitermaterial so aufgebracht wird, dass das Material molekular an dem Pelz-, Haut- oder Federartikel haftet.

3. Verfahren nach Anspruch 1 oder 2, wobei das Halbleitermaterial eine Talentartung von gv = 2 hat.

4. Verfahren nach einem der Ansprüche 1-3, wobei das Halbleitermaterial eine Elektronenbeweglichkeit von mehr als 15.000 cm² × V⁻¹ × s⁻¹ hat.

5. Verfahren nach einem der Ansprüche 1-4, wobei das Halbleitermaterial einen spezifischen Widerstand von weniger als 10⁻⁶ Ohm-cm hat.

6. Verfahren nach einem der Ansprüche 1-5, wobei das Halbleitermaterial Graphen ist.

7. Verfahren nach einem der Ansprüche 1-6, wobei das Halbleitermaterial als eine Klebstoffformulierung aufgebracht wird.

8. Chemisch behandelter Pelz-, Haut- oder Federartikel, aufweisend:
ein Pelz-, Haut- oder Federartikel; und
ein Halbleitermaterial, das molekular an einem Teil des Pelz-, Haut- oder Federartikels haftet, wodurch eine Schutzschicht gegen chemische Behandlungen bereitgestellt wird und ein geschützter Teil des Pelz-, Haut- oder Federartikels definiert wird.

9. Behandelter Pelz-, Haut- oder Federartikel nach Anspruch 8, wobei das Halbleitermaterial ein Null-Lücken-Halbleiter ist.

10. Behandelter Pelz-, Haut- oder Federartikel nach Anspruch 8 oder 9, wobei das Halbleitermaterial eine Talentartung von gv = 2 hat.

11. Behandelter Pelz-, Haut- oder Federartikel nach einem der Ansprüche 8-10, wobei das Halbleitermaterial eine Elektronenbeweglichkeit von mehr als 15.000 cm² × V⁻¹ × s⁻¹ hat.

12. Behandelter Pelz-, Haut- oder Federartikel nach einem der Ansprüche 8-11, wobei das Halbleitermaterial einen spezifischen Widerstand von weniger als 10⁻⁶ Ohm-cm hat.

13. Behandelter Pelz-, Haut- oder Federartikel nach einem der Ansprüche 8-12, wobei das Halbleitermaterial darauf gespeicherte digitale Informationen enthält.

## Revendications

1. Procédé de test d'ADN d'un produit de fourrure, de peau ou de plume, le procédé consistant à :
appliquer un matériau semi-conducteur sur une portion d'un produit de fourrure, de peau ou de plume, formant ainsi une couche protective contre les traitements chimiques et définissant ainsi une portion protégée du produit de fourrure, de peau ou de plume ;
traiter chimiquement le produit de fourrure, de peau ou de plume ;
retirer le matériau semi-conducteur de la portion protégée du produit de fourrure, de peau ou de plume ;
extraire l'ADN de la portion protégée du produit de fourrure, de peau ou de plume ; et
tester l'ADN extrait.

2. Procédé selon la revendication 1, dans lequel le matériau semi-conducteur est appliqué de telle sorte que le matériau adhère moléculairement au produit de fourrure, de peau ou de plume.

3. Procédé selon la revendication 1 ou 2, dans lequel le matériau semi-conducteur présente une dégénérescence de vallée, gv, égale à 2.

4. Procédé selon l'une quelconque des revendications 1 à 3, dans lequel le matériau semi-conducteur a une mobilité électronique supérieure à 15000 cm² × V⁻¹ × s⁻¹.

5. Procédé selon l'une quelconque des revendications 1 à 4, dans lequel le matériau semi-conducteur a une résistivité inférieure à 10⁻⁶ ohm centimètre.

6. Procédé selon l'une quelconque des revendications 1 à 5, dans lequel le matériau semi-conducteur est du graphène.

7. Procédé selon l'une quelconque des revendications 1 à 6, dans lequel le matériau semi-conducteur est appliqué sous la forme d'une formulation adhésive.

8. Produit traité chimiquement de fourrure, de peau ou de plume, comprenant :
un produit de fourrure, de peau ou de plume ; et
un matériau semi-conducteur ayant adhéré moléculairement à une portion du produit de fourrure, de peau ou de plume, fournissant ainsi une couche protective contre les traitements chimiques et définissant ainsi une portion protégée du produit de fourrure, de peau ou de plume.

9. Produit traité de fourrure, de peau ou de plume selon la revendication 8, dans lequel le matériau semi-conducteur est un semi-conducteur dont le gap est nul.

10. Produit traité de fourrure, de peau ou de plume selon la revendication 8 ou 9, dans lequel le matériau semi-conducteur présente une dégénérescence de vallée, gv, égale à 2.

11. Produit traité de fourrure, de peau ou de plume selon l'une quelconque des revendications 8 à 10, dans lequel le matériau semi-conducteur a une mobilité électronique supérieure à 15000 cm² × V⁻¹ × s⁻¹.

12. Produit traité de fourrure, de peau ou de plume selon l'une quelconque des revendications 8 à 11, dans lequel le matériau semi-conducteur a une résistivité inférieure à 10⁻⁶ ohm centimètre.

13. Produit traité de fourrure, de peau ou de plume selon l'une quelconque des revendications 8 à 12, dans lequel le matériau semi-conducteur inclut des informations numériques qui sont stockées dessus.
